# EUROPEAN PATENT APPLICATION

(11) **EP 3 513 796 A1**
(43) Date of publication of application: **24.07.2019**
(21) Application number: 17850921.2
(22) Date of filing: 13.09.2017
(51) Int. Cl.: A61K 31/706, A61P 25/20

(54) **SLEEP DISORDER IMPROVEMENT AGENT AND METHOD FOR IMPROVING SLEEP DISORDERS**

(30) Priority: 13.09.2016 JP 2016178764
(71) Applicant: Tanaka, Megumi, Kanagawa 253-0054 (JP); Tanaka, Tsunemaru, Odawara-shi, Kanagawa 256-0815 (JP)
(72) Inventor: Tanaka, Megumi, Kanagawa 253-0054 (JP); Tanaka, Tsunemaru, Odawara-shi, Kanagawa 256-0815 (JP)
(74) Representative: Müller & Schubert
(86) International application number: PCT/JP2017/033025
(87) International publication number: WO 2018/052020

(57) **Abstract**

PROBLEM: To provide an improving agent and an improving method for a sleep disorder that is safe in long-term intake and ensures effective improvement of the sleep disorder.

SOLUTION: Nicotinamide mononucleotide is contained as an active ingredient.

## Description

### TECHNICAL FIELD

The present invention relates to a sleep disorder improving agent and a method for improving the sleep disorder.

### BACKGROUND ART

A sleep has a function to maintain a body and a mind in normal conditions, and is one of important elements to keep health with, for example, a moderate exercise and a balanced eating habit. A study shows that disordered sleep causes troubles in, for example, recovery from fatigue of the body, fixation of memory, learning ability, release of stress, immune functions, emotional control, work ability, and judgement. Especially for children, the disordered sleep inhibits growth and development and causes poor school performance, emotional instability, and restlessness. Accordingly, comfortable and satisfactory sleep is significantly important for maintaining health. However, since the sleep is sensitively influenced by various matters such as a biorhythm, an external environment (for example, heat, cold, and noise level), mental effects (for example, stress and anxiety), and physical effects (for example, pain, itch, and disease), the sleep disorder such as insomnia easily occurs. Therefore, in modern society, people having troubles in sleep disorder or people having any problems on sleep, regardless of degree, rapidly increase at present.

A recent study points out, for example, an inappropriate life style, an excessive stress, and aging, which are distinctive phenomena in modern society, as factors to promote the occurrence of the sleep disorder. The inappropriate life style includes, for example, irregular bedtime and wake-up time, disturbance in eating habits, lack of exercise, coffee before falling asleep, high intake of tea, heavy drinking, reduced daytime activity, and nocturnal life. With development of an information-driven society and globalization, people in the inappropriate life style tend to increase.

In some diseases, strong relationships between sleep and onset have been known. For example, for an acute myocardial infarction, it has been confirmed that the diurnal variation is found in its onset frequency, and the onset frequency of the acute myocardial infarction is considered to increase after waking up. It has been known that a hormone secretory activity of a growth hormone, prolactin, and similar hormone has sleep dependence increasing or decreasing with direct relation with the sleep, and the sleep disorder causes endocrine diseases. Furthermore, it has been known that a gastric acid secretion closely relates to the sleep, and an onset of a peptic ulcer has a relation with the sleep. In addition, the disease strongly related with the sleep includes, for example, a cerebrovascular accident, a bronchial asthma, a high blood pressure, and a depression. Accordingly, it can be said that the satisfactory sleep is very important for the improvement and the prevention of these diseases.

The sleep disorder not only causes an abnormality on an individual body and mind to deteriorate the quality of the individual life, but also significantly influences a society and an economy of a nation. For example, an excessive sleepiness decreases attention and concentration, a work efficiency, and a willingness to work to impair productivity, and additionally, an excessive sleepiness causes increase in incidence of traffic accidents and accidents during work.

Thus, the sleep disorder not only worsens the health of the individual but also leads to damaging the safety and stability of the society and the economy of the nation. Accordingly, the improvement of the sleep disorder closely relates to the health of the individual and the stability and prosperity of the society, and thus it can be said to be an important problem to be immediately solved.

Therefore, currently, a topic regarding the improvement of the sleep disorder attracts much attention, and the studies and developments are popularly performed. Then, the results of those studies and developments have shown non-pharmacological treatments for the satisfactory sleep: promoting secretion of melatonin known as a sleep hormone and ensuring a regularity of the biorhythm; satisfactory wakefulness during daytime and before going to bed; arrangement of the satisfactory sleep environment; and relaxed state before going to bed provided by the regular eating habit, regular sleep schedule, regular light exercise, and sunbathing.

Meanwhile, conventionally, various sleeping pills have been used as pharmacological treatments against the sleep disorder such as the insomnia. As the sleeping pills, benzodiazepines (for example, nitrazepam, flurazepam, estazolam, nimetazolam, haloxazolam, triazolam, quazepam), thienodiazepines (for example, etizolam, protizolam), cyclopyrrolones (zopiclone), imidazopyridines (for example, zolpidem), barbituric acids (for example, phenobarbital, amobarbital, pentobarbital, secobarbital), non barbituric acids (for example, bromvalerylurea, butoctamide) are widely known, and an appropriate one is selected as necessary in accordance with the work period, the symptom, and the like.

In addition, as the conventional technique regarding the improvement of the sleep disorder, a sleep improving agent that contains a hot water extract of a microorganism of a cordyceps sinensis as an active ingredient is disclosed (Patent Document 1).

Regarding the improvement of especially a REM sleep disorder among the sleep disorders, a REM sleep disorder improving agent that contains N-acetyl-D-mannosamine is disclosed (Patent Document 2).

As a food product to improve the sleep disorder, a deep sleep disorder improving agent that contains glycine of 0.5 g or more as an intake per meal is disclosed (Patent Document 3).

Patent Document 1: JP-A-2010-53098
Patent Document 2: JP-A-2011-178702
Patent Document 3: JP-B-4913410

### DISCLOSURE OF THE INVENTION

### PROBLEMS TO BE SOLVED BY THE INVENTION

While the above-described non-pharmacological treatment is considered effective for improving the sleep disorder, a problem arises in that it is not necessarily easy to strictly keep and perform the above-described matters on the sleep hygiene. Meanwhile, the above-described pharmacological treatment has a problem where, for example, a drug resistance due to the long-term use, a rebound insomnia and a withdrawal symptom when the intake is stopped, dependency formation, a transient amnesia, sleepiness after awakening, and a nightmare are easily caused.

It is an object of the present invention to provide an improving agent and an improving method for a sleep disorder that is safe in long-term intake and ensures effective improvement of the sleep disorder.

### SOLUTIONS TO THE PROBLEMS

The inventor seriously studied in order to solve the above-described problems, and found that a nicotinamide mononucleotide as an intermediate metabolite involved in biosynthesis of a coenzyme NAD (nicotinamide adenine dinucleotide) has an excellent sleep disorder improving effect. Thus, the present invention was completed.

The present invention is as follows.
[1] A sleep disorder improving agent that contains a nicotinamide mononucleotide as an active ingredient.
[2] The sleep disorder improving agent according to [1] where the improvement of the sleep disorder is an improvement of insomnia.
[3] The sleep disorder improving agent according to [1] or [2] where the sleep disorder improving agent is a food product for improving the sleep disorder.
[4] The sleep disorder improving agent according to [1] or [2] where the sleep disorder improving agent is a medicinal product for improving the sleep disorder.
[5] A method for improving a sleep disorder that includes causing a target to ingest a nicotinamide mononucleotide by an effective dose, the target needs the nicotinamide mononucleotide by the effective dose (excluding a medical practice to a human).

### EFFECTS OF THE INVENTION

The present invention ensures the effective improvement of the sleep disorder. The present invention is safe because the nicotinamide mononucleotide as the intermediate metabolite involved in the biosynthesis of in vivo NAD⁺ is contained as the active ingredient. The present invention ensures the long-term intake.

### BRIEF DESCRIPTION OF THE DRAWINGS

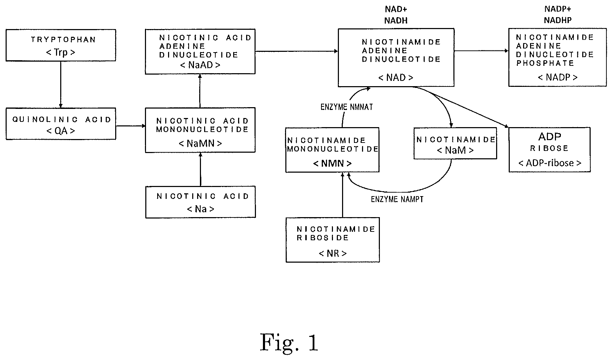

Fig. 1 is an explanatory drawing illustrating a metabolic pathway involved in niacin (generic term of nicotinamide and nicotinic acid).

### DESCRIPTION OF PREFERRED EMBODIMENTS

The sleep disorder improving agent according to the present invention contains the nicotinamide mononucleotide as the active ingredient and provides the improving effect of the sleep disorder. In the present invention, the improvement of the sleep disorder includes prevention, and stopping and delaying progress of the sleep disorder in addition to the improvement of the sleep disorder in narrow sense. The detailed reason why containing the nicotinamide mononucleotide as the active ingredient provides such an effect is currently examined. However, it is considered to be one reason that the nicotinamide mononucleotide promotes a "sirtuin" typified by NAD⁺ dependent deacetylases Sirt1 and Sirt3, and consequently, a glucose tolerance and hormone secretion systems of the growth hormone, cortisol, melatonin and similar hormone are normalized. The present invention is presumed to be based on a new mechanism of action completely different from benzodiazepine drugs and barbituric acid drugs, widely known as the conventional sleep disorder improving agents, that acts on a γ-aminobutyric acid (GABA) nervous system to improve the sleep disorder. The following describes the present invention in detail.

The nicotinamide mononucleotide (chemical formula: C₁₁H₁₅N₂O₈P) is a compound produced in bodies of many organisms including human, and expressed with a structural formula [Chem. 1] below. The nicotinamide mononucleotide is generally referred to as NMN, and known as an intermediate metabolite involved in a biosynthesis of coenzyme NAD⁺.

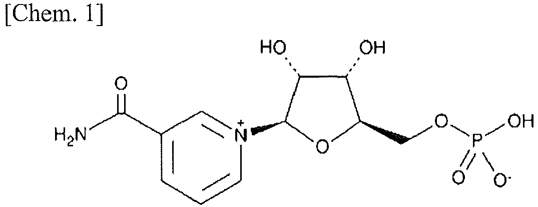

The nicotinamide mononucleotide as the active ingredient of the sleep disorder improving agent is produced in an NAD metabolic pathway by liver tissues, that is, a pathway involved in a synthesis of a nicotinamide adenine dinucleotide (NAD) from a quinolinic acid through a kynurenine pathway, in vivo. This will be specifically described with reference to Fig. 1. Fig. 1 is an explanatory drawing illustrating a metabolic pathway involved in niacin (generic term of a nicotinamide and a nicotinic acid) known as vitamin B₃. The nicotinic acid ingested through a meal is absorbed by the liver to be converted into nicotinamide, and the nicotinamide is supplied to the whole body via a blood flow. The cells each absorb the nicotinamide from the blood, and convert it into the NAD and an NADP to use them. The nicotinamide is biosynthesized also from a tryptophan.

As illustrated in Fig. 1, in vivo, when the tryptophan is a starting material, the tryptophan is converted into the quinolinic acid (QA) through the kynurenine pathway as a tryptophan metabolic pathway, and further converted into a nicotinic acid mononucleotide (NaMN). Meanwhile, when the nicotinic acid (Na) is the starting material, the nicotinic acid is directly converted into the NaMN. Afterwards, the NaMN is interconverted into the NAD, a nicotinamide (NaM), and the nicotinamide mononucleotide in a NAD cycle through a nicotinic acid adenine dinucleotide (NaAD). The nicotinamide (NaM) is converted into the nicotinamide mononucleotide by a nicotinamide phosphoribosyltransferase (NAMPT), subsequently, the nicotinamide mononucleotide is converted by a nicotinamide mononucleotide adenyltransferase (NMNAT) to generate the NAD. Note that, the nicotinamide mononucleotide is produced also from a nicotinamide riboside (NR) as an NAD intermediate metabolite.

The nicotinamide mononucleotide includes two types of an α-form and a β-form as optical isomers, and the β-form is used in the present invention. The nicotinamide mononucleotide is obtained by, for example, synthesizing a nicotinamide riboside from the nicotinamide and a ribose (see Bioorg. Med. Chem. Lett., 12, 1135-1137 (2002)), and subsequently, phosphorylating a 5-hydroxyl group of the ribose part (see Chem. Comm., 1999, 729-730). Specifically, for example, first, a reaction solution is prepared by dissolving the nicotinamide and an L-ribose tetraacetate in anhydrous acetonitrile, adding a trimethylsilyl trifluorosulfonic acid by an excessive amount under a nitrogen stream and then stirring at room temperature, and adding methanol to stop the reaction. The above-described reaction solution is poured into a column filled with activated carbon, cleaned with a distilled water, and then eluted with methanol and its product is collected. Next, for a phosphorylation reaction of the 5-hydroxyl group of the L-ribose part of this product, a reaction solution is prepared by dissolving the above-described product in a trimethoxy phosphoric acid, dropping a phosphorus oxychloride below freezing and stirring under the nitrogen stream, adding a sodium hydroxide aqueous solution to neutralize, thus stopping the reaction. A cold acetonitrile-ether solution is added to the above-described reaction solution. Afterwards, a lower layer (water phase) is passed through an anion-exchange resin to collect a reactant, and further purifies the reactant with a cation-exchange resin, thus the high-purity nicotinamide mononucleotide can be collected. The nicotinamide mononucleotide is commercially available from Oriental Yeast Co., ltd. and Bontac Bio-engineering (Shenzhen) Co., Ltd., and those commercial products can be purchased for use.

The nicotinamide mononucleotide is a purified product that contains a few impurities, especially, preferably its purity is 90% or more, and further preferably its purity is 95% or more. When the purity is 90% or less, a bad smell possibly occurs, or the effect of the nicotinamide mononucleotide is possibly reduced to fail to sufficiently provide the effect of the present invention.

While the purity of the nicotinamide mononucleotide is preferably 90% or more as described above, the purity (mass ratio) is defined as a value obtained by subtracting the impurities other than the nicotinamide mononucleotide from 100% in anhydrous terms. Accordingly, the purity of the nicotinamide mononucleotide can be obtained with a formula: nicotinamide mononucleotide purity (%) = 100 - impurities other than nicotinamide mononucleotide (%). Here, these impurities include, as illustrated in Fig. 1, metabolites excluding the nicotinamide mononucleotide involved in the NAD metabolic pathway, especially, the nicotinamide and the nicotinamide adenine dinucleotide. When the nicotinamide mononucleotide used in the present invention contains a foreign element such as the above-described metabolite involved in the NAD metabolic pathway, for example, the absorption of the nicotinamide mononucleotide into living cells possibly reduces, resulting in the reduction of the effect of the present invention. A quantitative determination of the above-described impurities involved in the NAD metabolic pathway is performed with an absolute calibration curve method using a standard sample where a test solution of dried nicotinamide mononucleotide powder is poured into an HPLC apparatus, and a peak area of an obtained chromatograph is obtained (vertical axis: peak area, horizontal axis: concentration). Since the use of the peak height ensures the quantitative determination with high accuracy in a case of a trace substance, the apparatus to be used is appropriately chosen according to the characteristics of the apparatus. Separated substances are identified based on retention times.

The sleep disorder improving agent according to the present invention is easily manufactured by using the nicotinamide mononucleotide alone or mixing another ingredient. The other ingredient is not specifically limited insofar as the effect of the present invention is provided.

The other ingredient includes, for example, anthocyanin, lutein, docosahexaenoic acid, astaxanthin, lycopene, taurine, panthenol, potassium aspartate, chondroitin sulfate, zinc, calcium, and magnesium, which are known ingredients said to have the sleep disorder improving effect. The other ingredient may include, for example, various kinds of vitamins, a trace element, citric acid, malic acid, a perfume, and an inorganic salt, which are auxiliary ingredients commonly used in the food field.

In the present invention, the other ingredient especially effective in increasing the sleep disorder improving effect includes resveratrol. The resveratrol is known as an antioxidant substance contained in, for example, a grape peel, red wine, a peanut peel, a Japanese knotweed, and a gnetum gnemon. The resveratrol includes trans and cis isomers, a trans/cis isomer mixture, a dimer, and a resveratrol derivative such as methylated resveratrol. The trans isomer stable against heat is usually used for the health food and the like. The resveratrol may be synthetically prepared in addition to one prepared by being extracted from every raw material and purified.

A compounding ratio of the resveratrol and the nicotinamide mononucleotide is not limited. However, from an aspect of extracting the maximum effect of the present invention, the compounding ratio of both is preferably adjusted such that in a daily intake per adult, the resveratrol is 1 to 100 pts.mass while the nicotinamide mononucleotide is 1 to 25 pts.mass.

The sleep disorder improving agent according to the present invention is mainly orally ingested to ensure the improvement of the sleep disorder. In the present invention, the sleep disorder includes the insomnia, a hypersomnia, a parasomnia, a sleep-related breathing disorder, a sleep-related dyskinesia, and similar symptom. Especially for the improvement of the insomnia, the sleep disorder improving agent according to the present invention is mainly orally ingested.

The insomnia is a disease of most frequent type among the sleep disorders, and the symptom includes a sleep-onset insomnia (hard to get to sleep), a difficulty in deep-sleep (light sleep), a sleep maintenance insomnia (wake up three times or more a night), an early morning awakening (wake up earlier than a desired time for two hours or more).

The hypersomnia means a state where strong sleepiness occurs in the daytime to cause a difficulty in staying awake regardless of the sufficient sleep in the nighttime. The hypersomnia is usually categorized in narcolepsy (having frequently repeated sleepiness), an idiopathich hypersomnia (night sleep is prolonged to cause difficulty in waking up in the morning and persistent sleepiness is felt in the daytime), and a recurrent hypersomnia (a time where the strong sleepiness is felt (somnolence period) continues for three days to three weeks, the symptom naturally recovers and is completely removed, but afterwards the somnolence period repeatedly appears at inconstant intervals).

The parasomnia is a generic term of undesirable physical phenomena that occur during sleep. The disease regarding the non-REM sleep includes a sleep inertia, a sleepwalking, a night terror, and similar disease. The disease regarding the REM sleep includes a REM sleep behavior disorder, a recurrent isolated sleep paralysis, a nightmare disorder, and similar disease.

The sleep-related breathing disorder is a disorder where a breathing disorder during sleep causes deterioration of sleep quality, and represented by an obstructive sleep apnea syndrome. The symptom of the obstructive sleep apnea syndrome includes, for example, stop breathing during sleep, snore, frequent waking during nighttime, early morning headache, daytime sleepiness, decreased concentration, and frustration.

The sleep-related dyskinesia is a sleep disorder caused by an involuntary movement occurred during sleep, and represented by a restless legs syndrome (dysesthesia in the lower limbs) and a periodic limb movement disorder (periodic occurrence of the involuntary movement).

The method for manufacturing the sleep disorder improving agent is not specifically limited, but a common manufacturing method used for manufacturing it may be appropriately chosen corresponding to its form. For example, when the form is powder, the sleep disorder improving agent can be manufactured by uniformly mixing the nicotinamide mononucleotide and the other ingredient contained as necessary. The nicotinamide mononucleotide as the active ingredient is distributed in the market and commercially available. Especially, for the nicotinamide mononucleotide, a quality management system and a mass production system of the nicotinamide mononucleotide are recently established.

The sleep disorder improving agent according to the present invention is usable as a food product and a medicinal product. In the case of the use as the food product, the sleep disorder improving agent can be provided as the food product for improving the sleep disorder in the food field. Daily ingestion in the form of the food product continuously provides the sleep disorder improving effect, thus being especially effective in improving the sleep disorder. The type of the food product as the target of the present invention is not specifically limited, and the target includes a functional food, a food for specified health use, a dietary supplement, a food additive, a feed, a care food, a diet therapy food, a therapeutic diet, a diet food, and similar food product in addition to general food products. Specifically, for example, confectionery (gum, candies, cookies, gummi candies, biscuits, cakes, chocolates, Japanese confectionery, jelly, and the like), bread, noodles, rice/grain processed foods (cereals and the like), meat processed foods, fish and shellfish processed foods, vegetable processed foods, ready-prepared foods, fermented foods, seasonings (source, dressing, ketchup, and the like), spices, dairy products (yogurt, cheese, milk, and the like), ice cream, frozen foods, retort pouch foods, beverages (carbonated beverages, soft drinks, milk-based beverages, alcoholic beverages, sports beverages, fruit-flavored beverages, teas, nutritious beverages, concentrated beverages, and the like), powdered beverages (powdered juice, powdered soup, and the like) are exemplified. The form of the food product is not limited, and especially in the case of the functional food, the food for specified health use, and the like, the food product can be processed to be provided in the form of, for example, a powder, a tablet, a pill, a granule, a hard capsule formulation, a soft capsule formulation, a jelly, a liquid medicine, and a paste medicine.

The intake of the food product is different depending on the type of the food product, age, sex, and weight of a target that takes the food product, the expected effect, and the symptom. However, the daily intake per adult of the nicotinamide mononucleotide contained in the food product is ordinarily 1 mg to 500 mg, preferably 5 mg to 300 mg, and more preferably 50 mg to 300 mg. Less than 1 mg possibly fails to provide the effect of the present invention, while more than 500 mg merely provides almost similar effect but causes economic disadvantage. The compounding ratio of the nicotinamide mononucleotide in the food product can be appropriately set relative to a total food weight in a range of 100% or less.

The food product is safe and side effects are not specifically recognized. Therefore, the food product can be ingested over a long period of time not only to improve the sleep disorder but also to prevent the sleep disorder. Accordingly, the food product is applicable to not only the target desired to improve the sleep disorder but also the healthy target so as not to cause the sleep disorder.

Meanwhile, the sleep disorder improving agent according to the present invention can be administered orally or non-orally as a medicinal product (including quasi-drugs) for the improvement of the sleep disorder in the pharmaceutical field. A dosage form of the medicinal product is not specifically limited, but can include, for example, a powder, a tablet, a persistent tablet, a chewable tablet, an effervescent tablet, a troche, a buccal tablet, a sublingual tablet, a capsule formulation, a fine granule, a granule, a pill, a dry syrup, a liquid medicine, a suspending agent, a syrup, a formulation for oral administration such as an elixir, and an eye drop, an eyewash, an eye ointment, an injection preparation, a transfusion, and an external preparation. Among these forms, considering the ease of taking, the stability of the active ingredient, and the like, the formulation for oral administration such as the powder, the tablet, and the capsule formulation is preferable.

The medicinal product can appropriately contain a known additive for formulation, which is adequate for the dosage form and pharmacologically allowed, considering physicochemical property, biological property, and similar property. Such an additive for formulation is exemplified by, for example, an excipient (lactose, starch, crystalline cellulose, sodium phosphate, and the like), a solvent (water, soybean oil, saline solution, a nonaqueous solvent for injection, and the like), a binder (starch, gelatin, gum arabic, sodium alginate, carmellose sodium, methylcellulose, ethyl cellulose, hydroxypropyl methylcellulose, hydroxypropyl cellulose, polyvinylpyrrolidone, and the like), a disintegrant (starch, carmellose sodium, and the like), a lubricant (talc, magnesium stearate, calcium stearate, macrogol, sucrose fatty acid ester, and the like), a coating agent (white sugar, HPC, shellac, gelatin, glycerin, hydroxypropyl methylcellulose, hydroxypropyl methylcellulose phthalate, cellulose acetate phthalate, and the like), a stabilizer (sodium bisulfite, sodium thiosulfate, sodium edetate, sodium citrate, ascorbic acid, dibutylhydroxytoluene, and the like), a preservative (methyl parahydroxybenzoate, ethyl parahydroxybenzoate, propyl parahydroxybenzoate, benzyl alcohol, phenol, chlorobutanol, benzalkonium chloride, benzethonium chloride, sodium dehydroacetate, thimerosal, and the like), a viscous agent (methylcellulose, carmellose sodium, chondroitin sulfate, sodium alginate, and the like), a suspending agent (various nonionic surfactant, methylcellulose, carmellose sodium, and the like), an emulsifier (gum arabic, cholesterol, sorbitan sesquioleate, polysorbate 80, sodium lauryl sulfate, and the like), a buffer (citric acid, acetic acid, sodium phosphate, and boric acid), a surfactant (hydrogenated castor oil, polysorbate 80, and the like), a colorant (water-soluble food pigment, lake pigment, and the like), a corrigent (lactose, white sugar, glucose, mannitol, and the like), a scenting agent (aromatic essential oils), a plasticizer (the phthalic acid esters, vegetable oils, polyethylene glycol, and the like).

A dose of the medicinal product cannot be equally specified because it differs depending on, for example, age, weight, and symptom of the administration target and the number of times of the administration. However, as the dose of the medicinal product, the daily amount per adult of the nicotinamide mononucleotide to be administered is ordinarily 1 mg to 500 mg, preferably 5 mg to 300 mg, and more preferably 50 mg to 300 mg. Less than 1 mg possibly fails to provide the effect of the present invention, while more than 500 mg merely provides almost similar effect but causes economic disadvantage. The compounding ratio of the nicotinamide mononucleotide in the medicinal product can be appropriately set in accordance with the dosage form, the dose of the medicinal product, and the like.

The number of times of the administration of the medicinal product can be appropriately set in accordance with, for example, the age, weight, and symptom of the administration target and the dose per administration of the medicinal product. The number of times of the medicinal product administration per day can be exemplified by once to three times.

Since the nicotinamide mononucleotide has the sleep disorder improving effect as described above, the present invention further provides a method for improving the sleep disorder to cause a target that needs an effective dose of the nicotinamide mononucleotide to ingest it. That is, a method for improving the sleep disorder of the target of the ingestion by causing the target to ingest the sleep disorder improving agent according to the present invention. The target of the ingestion is preferably a mammal such as a human, a mouse, a rat, a rabbit, a dog, a cat, cattle, a horse, a pig, and a monkey, and especially a human is preferable. In the method, the intake of the nicotinamide mononucleotide, the number of intake per day, and similar matters are as described for the sleep disorder improving agent. The sleep disorder improving agent can be ingested anytime in any case, and can be ingested by the target over a long period of time.

### [Working Example]

The following describes the present invention in detail based on the working examples, while the present invention is not limited by these working examples.

### Production Example 1

Commercially available nicotinamide mononucleotide was uniformly mixed with starch, filled in hard capsules, and capsule formulations containing the nicotinamide mononucleotide of 25 mg and the starch of 320 mg per capsule were manufactured.

### Production Example 2

Commercially available nicotinamide mononucleotide was uniformly mixed with starch, filled in hard capsules, and capsule formulations containing the nicotinamide mononucleotide of 50 mg and the starch of 292 mg per capsule were manufactured.

### Production Example 3

Commercially available nicotinamide mononucleotide was uniformly mixed with starch, filled in hard capsules, and capsule formulations containing the nicotinamide mononucleotide of 100 mg and the starch of 195 mg per capsule were manufactured.

### Production Example 4

Commercially available nicotinamide mononucleotide was uniformly mixed with starch, filled in hard capsules, and capsule formulations containing the nicotinamide mononucleotide of 150 mg and the starch of 145 mg per capsule were manufactured.

### Production Example 5

With a usual method, ingredients such as commercially available nicotinamide mononucleotide and resveratrol described in a prescription below were uniformly mixed, filled in hard capsules, and capsule formulations containing the nicotinamide mononucleotide of 5 mg and the resveratrol of 10 mg per capsule were manufactured based on the prescription below.

**Prescription**

| | |
|---|---|
| resveratrol | 10 mg |
| β-nicotinamide mononucleotide | 5 mg |
| pig placenta | 1 mg |
| collagen | 10 mg |
| hyaluronic acid | 0.125 mg |
| elastin | 0.013 mg |
| ceramide | 0.013 mg |
| peucedanum japonicum | 0.125 mg |
| vitamin C | 10 mg |
| amino acid mix | 1.25 mg |
| vitamin mix | 1.25 mg |
| starch | 201.225 mg |
| calcium stearate | 5 mg |
| fine silicon dioxide | 5 mg |
| sum | 250 mg |

### Working Example 1

14 subjects (7 males, 7 females, age 39 to 73) with the symptom of sleep-onset insomnia took the capsule formulation manufactured in Production Example 1 by two capsules a day for two months continuously. The subjects performed self-evaluations on the symptom of sleep-onset insomnia with criteria indicated in Table 1 at each of two weeks, one month, and two months after the start of taking. Table 1 indicates the result of the evaluated number of people for each criterion.

### Working Example 2

13 subjects (6 males, 7 females, age 39 to 73) with the symptom of light sleep took the capsule formulation manufactured in Production Example 1 by two capsules a day for two months continuously. The subjects performed self-evaluations on the symptom of light sleep with criteria indicated in Table 1 at each of two weeks, one month, and two months after the start of taking. Table 1 indicates the result of the evaluated number of people for each criterion.

### Working Example 3

Six subjects (4 males, 2 females, age 50 to 73) with the symptom of sleep maintenance insomnia took the capsule formulation manufactured in Production Example 1 by two capsules a day for two months continuously. The subjects performed self-evaluations on the symptom of sleep maintenance insomnia with criteria indicated in Table 1 at each of two weeks, one month, and two months after the start of taking. Table 1 indicates the result of the evaluated number of people for each criterion.

### Working Example 4

Six subjects (4 males, 2 females, age 48 to 73) with the symptom of early morning awakening took the capsule formulation manufactured in Production Example 1 by two capsules a day for two months continuously. The subjects performed self-evaluations on the symptom of early morning awakening with criteria indicated in Table 1 at each of two weeks, one month, and two months after the start of taking. Table 1 indicates the result of the evaluated number of people for each criterion.

### Working Example 5

Four subjects (2 males, 2 females, age 49 to 73) with the symptom of hypersomnia took the capsule formulation manufactured in Production Example 1 by two capsules a day for two months continuously. The subjects performed self-evaluations on the symptom of hypersomnia with criteria indicated in Table 1 at each of two weeks, one month, and two months after the start of taking. Table 1 indicates the result of the evaluated number of people for each criterion.

### Working Example 6

Seven subjects (7 males, 0 females, age 50 to 67) with the symptom of snore took the capsule formulation manufactured in Production Example 1 by two capsules a day for two months continuously. The subjects performed self-evaluations on the symptom of snore with criteria indicated in Table 1 at each of two weeks, one month, and two months after the start of taking. Table 1 indicates the result of the evaluated number of people for each criterion.

### Working Example 7

Three subjects (3 males, 0 females, age 63 to 67) with the symptom of dysesthesia in the lower limbs during sleep took the capsule formulation manufactured in Production Example 1 by two capsules a day for two months continuously. The subjects performed self-evaluations on the symptom of dysesthesia in the lower limbs during sleep with criteria indicated in Table 1 at each of two weeks, one month, and two months after the start of taking. Table 1 indicates the result of the evaluated number of people for each criterion.

**[Table 1]**

| | | Symptom got worse | Effect was not especially felt | Slightly improved | Improved | Considerably improved |
|---|---|---|---|---|---|---|
| Working Example 1 | Two weeks later | - | 5 | 9 | - | - |
| | One month later | - | 2 | 7 | 5 | - |
| | Two months later | - | 1 | 8 | 5 | - |
| Working Example 2 | Two weeks later | - | 3 | 7 | 3 | - |
| | One month later | - | 1 | 5 | 7 | - |
| | Two months later | - | - | 6 | 6 | 1 |
| Working Example 3 | Two weeks later | - | 3 | 2 | 1 | - |
| | One month later | - | 1 | 4 | 1 | - |
| | Two months later | - | - | 3 | 3 | - |
| Working Example 4 | Two weeks later | - | 4 | 2 | - | - |
| | One month later | - | 1 | 5 | - | - |
| | Two months later | - | - | 2 | 4 | - |
| Working Example 5 | Two weeks later | - | 1 | 3 | - | - |
| | One month later | - | - | 3 | 1 | - |
| | Two months later | - | - | 3 | 1 | - |
| Working Example 6 | Two weeks later | - | 7 | - | - | - |
| | One month later | - | 6 | 1 | - | - |
| | Two months later | - | 2 | 4 | 1 | - |
| Working Example 7 | Two weeks later | - | 3 | - | - | - |
| | One month later | - | 3 | - | - | - |
| | Two months later | - | 2 | 1 | - | - |

### Working Example 8

21 subjects (12 males, 9 females, age 38 to 78) with the symptom of sleep-onset insomnia took the capsule formulation manufactured in Production Example 2 by two capsules a day for two months continuously. The subjects performed self-evaluations on the symptom of sleep-onset insomnia with criteria indicated in Table 2 at each of two weeks, one month, and two months after the start of taking. Table 2 indicates the result of the evaluated number of people for each criterion.

### Working Example 9

21 subjects (12 males, 9 females, age 38 to 78) with the symptom of light sleep took the capsule formulation manufactured in Production Example 2 by two capsules a day for two months continuously. The subjects performed self-evaluations on the symptom of light sleep with criteria indicated in Table 2 at each of two weeks, one month, and two months after the start of taking. Table 2 indicates the result of the evaluated number of people for each criterion.

### Working Example 10

18 subjects (12 males, 6 females, age 38 to 78) with the symptom of sleep maintenance insomnia took the capsule formulation manufactured in Production Example 2 by two capsules a day for two months continuously. The subjects performed self-evaluations on the symptom of sleep maintenance insomnia with criteria indicated in Table 2 at each of two weeks, one month, and two months after the start of taking. Table 2 indicates the result of the evaluated number of people for each criterion.

### Working Example 11

18 subjects (12 males, 6 females, age 38 to 78) with the symptom of early morning awakening took the capsule formulation manufactured in Production Example 2 by two capsules a day for two months continuously. The subjects performed self-evaluations on the symptom of early morning awakening with criteria indicated in Table 2 at each of two weeks, one month, and two months after the start of taking. Table 2 indicates the result of the evaluated number of people for each criterion.

### Working Example 12

18 subjects (11 males, 7 females, age 38 to 78) with the symptom of hypersomnia took the capsule formulation manufactured in Production Example 2 by two capsules a day for two months continuously. The subjects performed self-evaluations on the symptom of hypersomnia with criteria indicated in Table 2 at each of two weeks, one month, and two months after the start of taking. Table 2 indicates the result of the evaluated number of people for each criterion.

### Working Example 13

14 subjects (10 males, 4 females, age 38 to 76) with the symptom of snore took the capsule formulation manufactured in Production Example 2 by two capsules a day for two months continuously. The subjects performed self-evaluations on the symptom of snore with criteria indicated in Table 2 at each of two weeks, one month, and two months after the start of taking. Table 2 indicates the result of the evaluated number of people for each criterion.

### Working Example 14

10 subjects (6 males, 4 females, age 48 to 76) with the symptom of dysesthesia in the lower limbs during sleep took the capsule formulation manufactured in Production Example 2 by two capsules a day for two months continuously. The subjects performed self-evaluations on the symptom of dysesthesia in the lower limbs during sleep with criteria indicated in Table 2 at each of two weeks, one month, and two months after the start of taking. Table 2 indicates the result of the evaluated number of people for each criterion.

### Working Example 15

Five subjects (2 males, 3 females, age 53 to 76) with the symptom of undesirable physical phenomena that occur during sleep (sleep inertia, sleepwalking, etc.) took the capsule formulation manufactured in Production Example 2 by two capsules a day for two months continuously. The subjects performed self-evaluations on the symptom of undesirable physical phenomena that occur during sleep (sleep inertia, sleepwalking, etc.) with criteria indicated in Table 2 at each of two weeks, one month, and two months after the start of taking. Table 2 indicates the result of the evaluated number of people for each criterion.

**[Table 2]**

| | | Symptom got worse | Effect was not especially felt | Slightly improved | Improved | Considerably improved |
|---|---|---|---|---|---|---|
| Working Example 8 | Two weeks later | - | 4 | 13 | 2 | 2 |
| | One month later | - | - | 9 | 10 | 2 |
| | Two months later | - | - | 3 | 15 | 3 |
| Working Example 9 | Two weeks later | - | 2 | 15 | 2 | 2 |
| | One month later | - | - | 10 | 9 | 2 |
| | Two months later | - | - | 6 | 11 | 4 |
| Working Example 10 | Two weeks later | - | 6 | 10 | - | 2 |
| | One month later | - | 2 | 9 | 5 | 2 |
| | Two months later | - | 2 | 6 | 7 | 3 |
| Working Example 11 | Two weeks later | - | 6 | 10 | - | 2 |
| | One month later | - | 4 | 10 | 2 | 2 |
| | Two months later | - | 3 | 6 | 7 | 2 |
| Working Example 12 | Two weeks later | - | 11 | 5 | - | 2 |
| | One month later | - | 6 | 10 | - | 2 |
| | Two months later | - | 4 | 9 | 3 | 2 |
| Working Example 13 | Two weeks later | - | 11 | 3 | - | - |
| | One month later | - | 7 | 6 | 1 | - |
| | Two months later | - | 1 | 11 | 2 | - |
| Working Example 14 | Two weeks later | - | 7 | 3 | - | - |
| | One month later | - | 3 | 7 | - | - |
| | Two months later | - | 3 | 6 | 1 | - |
| Working Example 15 | Two weeks later | - | 5 | - | - | - |
| | One month later | - | 3 | 2 | - | - |
| | Two months later | - | 1 | 4 | - | - |

### Working Example 16

11 subjects (4 males, 7 females, age 42 to 84) with the symptom of sleep-onset insomnia took the capsule formulation manufactured in Production Example 3 by two capsules a day for two months continuously. The subjects performed self-evaluations on the symptom of sleep-onset insomnia with criteria indicated in Table 3 at each of two weeks, one month, and two months after the start of taking. Table 3 indicates the result of the evaluated number of people for each criterion.

### Working Example 17

Nine subjects (4 males, 5 females, age 42 to 74) with the symptom of light sleep took the capsule formulation manufactured in Production Example 3 by two capsules a day for two months continuously. The subjects performed self-evaluations on the symptom of light sleep with criteria indicated in Table 3 at each of two weeks, one month, and two months after the start of taking. Table 3 indicates the result of the evaluated number of people for each criterion.

### Working Example 18

Seven subjects (3 males, 4 females, age 44 to 84) with the symptom of sleep maintenance insomnia took the capsule formulation manufactured in Production Example 3 by two capsules a day for two months continuously. The subjects performed self-evaluations on the symptom of sleep maintenance insomnia with criteria indicated in Table 3 at each of two weeks, one month, and two months after the start of taking. Table 3 indicates the result of the evaluated number of people for each criterion.

### Working Example 19

10 subjects (4 males, 6 females, age 42 to 84) with the symptom of early morning awakening took the capsule formulation manufactured in Production Example 3 by two capsules a day for two months continuously. The subjects performed self-evaluations on the symptom of early morning awakening with criteria indicated in Table 3 at each of two weeks, one month, and two months after the start of taking. Table 3 indicates the result of the evaluated number of people for each criterion.

### Working Example 20

Eight subjects (4 males, 4 females, age 44 to 84) with the symptom of hypersomnia took the capsule formulation manufactured in Production Example 3 by two capsules a day for two months continuously. The subjects performed self-evaluations on the symptom of hypersomnia with criteria indicated in Table 3 at each of two weeks, one month, and two months after the start of taking. Table 3 indicates the result of the evaluated number of people for each criterion.

### Working Example 21

Seven subjects (4 males, 3 females, age 44 to 84) with the symptom of snore took the capsule formulation manufactured in Production Example 3 by two capsules a day for two months continuously. The subjects performed self-evaluations on the symptom of snore with criteria indicated in Table 3 at each of two weeks, one month, and two months after the start of taking. Table 3 indicates the result of the evaluated number of people for each criterion.

### Working Example 22

Seven subjects (2 males, 5 females, age 62 to 84) with the symptom of dysesthesia in the lower limbs during sleep took the capsule formulation manufactured in Production Example 3 by two capsules a day for two months continuously. The subjects performed self-evaluations on the symptom of dysesthesia in the lower limbs during sleep with criteria indicated in Table 3 at each of two weeks, one month, and two months after the start of taking. Table 3 indicates the result of the evaluated number of people for each criterion.

### Working Example 23

Five subjects (0 males, 5 females, age 62 to 84) with the symptom of undesirable physical phenomena that occur during sleep (sleep inertia, sleepwalking, etc.) took the capsule formulation manufactured in Production Example 3 by two capsules a day for two months continuously. The subjects performed self-evaluations on the symptom of undesirable physical phenomena that occur during sleep (sleep inertia, sleepwalking, etc.) with criteria indicated in Table 3 at each of two weeks, one month, and two months after the start of taking. Table 3 indicates the result of the evaluated number of people for each criterion.

**[Table 3]**

| | | Symptom got worse | Effect was not especially felt | Slightly improved | Improved | Considerably improved |
|---|---|---|---|---|---|---|
| Working Example 16 | Two weeks later | - | - | 7 | 4 | - |
| | One month later | - | - | 1 | 9 | 1 |
| | Two months later | - | - | 1 | 7 | 3 |
| Working Example 17 | Two weeks later | - | - | 7 | 2 | - |
| | One month later | - | - | 1 | 7 | 1 |
| | Two months later | - | - | 1 | 5 | 3 |
| Working Example 18 | Two weeks later | - | - | 5 | 2 | - |
| | One month later | - | - | 1 | 6 | - |
| | Two months later | - | - | 1 | 6 | - |
| Working Example 19 | Two weeks later | - | 1 | 7 | 2 | - |
| | One month later | - | - | 4 | 6 | - |
| | Two months later | - | - | 2 | 8 | - |
| Working Example 20 | Two weeks later | - | 3 | 5 | - | - |
| | One month later | - | 1 | 6 | 1 | - |
| | Two months later | - | - | 5 | 3 | - |
| Working Example 21 | Two weeks later | - | 1 | 6 | - | - |
| | One month later | - | 1 | 4 | 2 | - |
| | Two months later | - | 1 | 4 | 2 | - |
| Working Example 22 | Two weeks later | - | 5 | 2 | - | - |
| | One month later | - | 4 | 2 | 1 | - |
| | Two months later | - | 2 | 4 | 1 | - |
| Working Example 23 | Two weeks later | - | 3 | 2 | - | - |
| | One month later | - | 3 | 1 | 1 | - |
| | Two months later | - | 1 | 2 | 2 | - |

### Working Example 24

18 subjects (13 males, 5 females, age 42 to 82) with the symptom of sleep-onset insomnia took the capsule formulation manufactured in Production Example 4 by two capsules a day for two months continuously. The subjects performed self-evaluations on the symptom of sleep-onset insomnia with criteria indicated in Table 4 at each of two weeks, one month, and two months after the start of taking. Table 4 indicates the result of the evaluated number of people for each criterion.

### Working Example 25

18 subjects (13 males, 5 females, age 42 to 82) with the symptom of light sleep took the capsule formulation manufactured in Production Example 4 by two capsules a day for two months continuously. The subjects performed self-evaluations on the symptom of light sleep with criteria indicated in Table 4 at each of two weeks, one month, and two months after the start of taking. Table 4 indicates the result of the evaluated number of people for each criterion.

### Working Example 26

12 subjects (7 males, 5 females, age 42 to 82) with the symptom of sleep maintenance insomnia took the capsule formulation manufactured in Production Example 4 by two capsules a day for two months continuously. The subjects performed self-evaluations on the symptom of sleep maintenance insomnia with criteria indicated in Table 4 at each of two weeks, one month, and two months after the start of taking. Table 4 indicates the result of the evaluated number of people for each criterion.

### Working Example 27

14 subjects (11 males, 3 females, age 45 to 82) with the symptom of early morning awakening took the capsule formulation manufactured in Production Example 4 by two capsules a day for two months continuously. The subjects performed self-evaluations on the symptom of early morning awakening with criteria indicated in Table 4 at each of two weeks, one month, and two months after the start of taking. Table 4 indicates the result of the evaluated number of people for each criterion.

### Working Example 28

15 subjects (11 males, 4 females, age 45 to 82) with the symptom of hypersomnia took the capsule formulation manufactured in Production Example 4 by two capsules a day for two months continuously. The subjects performed self-evaluations on the symptom of hypersomnia with criteria indicated in Table 4 at each of two weeks, one month, and two months after the start of taking. Table 4 indicates the result of the evaluated number of people for each criterion.

### Working Example 29

15 subjects (13 males, 2 females, age 51 to 82) with the symptom of snore took the capsule formulation manufactured in Production Example 4 by two capsules a day for two months continuously. The subjects performed self-evaluations on the symptom of snore with criteria indicated in Table 4 at each of two weeks, one month, and two months after the start of taking. Table 4 indicates the result of the evaluated number of people for each criterion.

### Working Example 30

Nine subjects (7 males, 2 females, age 45 to 82) with the symptom of dysesthesia in the lower limbs during sleep took the capsule formulation manufactured in Production Example 4 by two capsules a day for two months continuously. The subjects performed self-evaluations on the symptom of dysesthesia in the lower limbs during sleep with criteria indicated in Table 4 at each of two weeks, one month, and two months after the start of taking. Table 4 indicates the result of the evaluated number of people for each criterion.

### Working Example 31

Eight subjects (7 males, 1 females, age 51 to 82) with the symptom of undesirable physical phenomena that occur during sleep (sleep inertia, sleepwalking, etc.) took the capsule formulation manufactured in Production Example 4 by two capsules a day for two months continuously. The subjects performed self-evaluations on the symptom of undesirable physical phenomena that occur during sleep (sleep inertia, sleepwalking, etc.) with criteria indicated in Table 4 at each of two weeks, one month, and two months after the start of taking. Table 4 indicates the result of the evaluated number of people for each criterion.

**[Table 4]**

| | | Symptom got worse | Effect was not especially felt | Slightly improved | Improved | Considerably improved |
|---|---|---|---|---|---|---|
| Working Example 24 | Two weeks later | - | - | 8 | 10 | - |
| | One month later | - | - | 1 | 10 | 7 |
| | Two months later | - | - | - | 6 | 12 |
| Working Example 25 | Two weeks later | - | - | 8 | 10 | - |
| | One month later | - | - | 1 | 13 | 4 |
| | Two months later | - | - | - | 9 | 9 |
| Working Example 26 | Two weeks later | - | 1 | 9 | 2 | - |
| | One month later | - | - | 4 | 8 | - |
| | Two months later | - | - | 2 | 8 | 2 |
| Working Example 27 | Two weeks later | - | 2 | 7 | 5 | - |
| | One month later | - | - | 4 | 9 | 1 |
| | Two months later | - | - | 2 | 10 | 2 |
| Working Example 28 | Two weeks later | - | 2 | 7 | 6 | - |
| | One month later | - | 1 | 3 | 11 | - |
| | Two months later | - | - | 1 | 14 | - |
| Working Example 29 | Two weeks later | - | 2 | 10 | 3 | - |
| | One month later | - | 1 | 7 | 7 | - |
| | Two months later | - | 1 | 3 | 11 | - |
| Working Example 30 | Two weeks later | - | 1 | 6 | 2 | - |
| | One month later | - | 1 | 4 | 4 | - |
| | Two months later | - | 1 | 3 | 5 | - |
| Working Example 31 | Two weeks later | - | 1 | 7 | - | - |
| | One month later | - | 1 | 4 | 3 | - |
| | Two months later | - | - | 4 | 4 | - |

### Working Example 32

Five subjects (3 males, 2 females, age 38 to 68) with the symptom of sleep-onset insomnia took the capsule formulation manufactured in Production Example 5 by two capsules a day for two months continuously. The subjects performed self-evaluations on the symptom of sleep-onset insomnia with criteria indicated in Table 5 at each of two weeks, one month, and two months after the start of taking. Table 5 indicates the result of the evaluated number of people for each criterion.

### Working Example 33

Five subjects (3 males, 2 females, age 38 to 68) with the symptom of light sleep took the capsule formulation manufactured in Production Example 5 by two capsules a day for two months continuously. The subjects performed self-evaluations on the symptom of light sleep with criteria indicated in Table 5 at each of two weeks, one month, and two months after the start of taking. Table 5 indicates the result of the evaluated number of people for each criterion.

**[Table 5]**

| | | Symptom got worse | Effect was not especially felt | Slightly improved | Improved | Considerably improved |
|---|---|---|---|---|---|---|
| Working Example 32 | Two weeks later | - | 3 | 2 | - | - |
| | One month later | - | 1 | 4 | - | - |
| | Two months later | - | 1 | 3 | 1 | - |
| Working Example 33 | Two weeks later | - | 4 | 1 | - | - |
| | One month later | - | 2 | 3 | - | - |
| | Two months later | - | 1 | 4 | - | - |

As seen from the above-described results, the proportion of the subjects provided with the improving effect of the sleep disorder ("slightly improved," "improved," and "considerably improved") by taking the sleep disorder improving agent of the present invention was considerably high, and this was observed to ensure the improving effect of the sleep disorder by the sleep disorder improving agent of the present invention.

## Claims

1. A sleep disorder improving agent that contains a nicotinamide mononucleotide as an active ingredient.

2. The sleep disorder improving agent according to claim 1, wherein
the improvement of the sleep disorder is an improvement of insomnia.

3. The sleep disorder improving agent according to claim 1 or 2, wherein
the sleep disorder improving agent is a food product for improving the sleep disorder.

4. The sleep disorder improving agent according to claim 1 or 2, wherein
the sleep disorder improving agent is a medicinal product for improving the sleep disorder.

5. A method for improving a sleep disorder, comprising
causing a target to ingest a nicotinamide mononucleotide by an effective dose, the target needing the nicotinamide mononucleotide by the effective dose (excluding a medical practice to a human).
